# EUROPEAN PATENT SPECIFICATION

(11) **EP 0 760 366 B1**
(45) Date of publication and mention of the grant of the patent: **26.01.2000**
(21) Application number: 96306174.2
(22) Date of filing: 23.08.1996
(51) Int. Cl.: C07C 405/00, A61K 31/557, A61K 47/48, A61K 9/127, C08B 37/16

(54) **Prostaglandin derivatives**
Prostaglandinderivate
Dérivés de prostaglandine

(30) Priority: 25.08.1995 JP 23898495
(43) Date of publication of application: 05.03.1997
(73) Proprietor: ONO PHARMACEUTICAL CO., LTD., Chuo-ku Osaka 541 (JP)
(72) Inventor: Nishiura, Akio, Ono Pharmac. Co., Mishima-gun, Osaka (JP); Seko, Takuya, Ono Pharmac. Co., Mishima-gun, Osaka (JP); Matsumoto, Ryoji, Ono Pharmac. Co., Mishima-gun, Osaka (JP); Hamano, Shin-ichi, Ono Pharmac. Co., Mishima-gun, Osaka (JP)
(74) Representative: Bentham, Stephen

(56) References cited:
- GB-A- 1 322 637
- US-A- 4 054 736

## Description

The present invention relates to prostaglandin E₁ (hereinafter abbreviated as PGE₁) ester derivatives or cyclodextrin clathrates thereof, liposome formulations containing them, processes for their preparation and pharmaceutical compositions containing them as active ingredient.

PGE₁ is represented by the following structure of the formula: 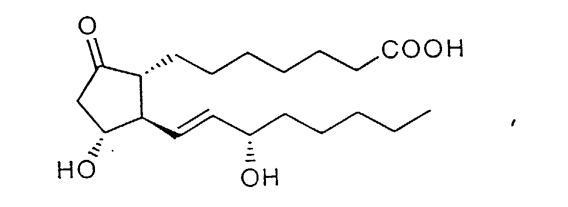 ,and has various physiological properties. In particular, PGE₁ has a hypotensive, vasodilatory, blood flow increasing and anti-platelet effect on blood vessels.

Because of its various physiological properties, PGE₁ has been applied in medicine. Already, PGE₁ has been used for treatment of peripheral arterial occlusive disease, thrombotic anginetic obliterence etc., for maintenance of blood flow after reconstructive vascular surgery, maintenance of low level of blood pressure at a surgical operation, as an anesthetic etc.

However, in order to use PGE₁ for improving peripheral circulatory disorder, there are two problems as follows:
(1) PGE₁ has many physiological properties. Therefore, if one physiological action of PGE₁ is applied to the therapy, other effects become, themselves, side effects.
(2) PGE₁ is rapidly inactivated by its metabolizing enzyme in vivo.

Peripheral circulatory disorder is a disease accompanied by various ischemic symptoms such as pain, psychroesthesia, etc., in which obstructions are induced by thrombus formation in peripheral blood vessels, and following ulcer formation. In order to treat this disorder, it is necessary to improve the blood circulation by increasing blood flow in the peripheral circulation.

However, if a large amount of PGE₁ is injected into the blood vessel at once, it acts not only on peripheral circulation but also on aortic series, therefore there is a fear of serious hypotension. In order to prevent this problem, PGE₁ should be injected in controlled doses so that it acts on the peripheral circulation, but acts to a lesser degree on aortic series.

On the other hand, it is known that PGE₁ is very rapidly metabolized. Accordingly, in order to maintain the effect, it is required that PGE₁ is sequentially administered in vivo. As a result of considering these effects in combination, it is desired to prepare a compound that is converted into PGE₁ in vivo after administering, and furthermore, the rate of the conversion of which is moderately slow, so that the effect is maintained.

The present inventors have investigated to discover a compound that is gradually converted into PGE₁ in vivo after administering. As a result, the present inventors have found that this result may be achieved with the compounds in which the carboxylic acid group at the 1-position of PGE₁ is esterified by a specific alcohol.

Further, the present inventors have also found that the effect may be improved by enclosing the compounds of the present invention with a closed vesicle comprising a phospholipid bi-layer called a liposome.

It is described in the specification of Great Britain Patent No. 1322637 that the compounds of the formula (B): 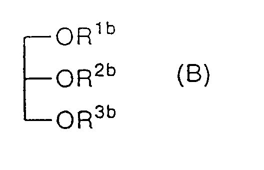 in which R^{1b}, R^{2b} and R^{3b} represent, inter alia, hydrogen, stearoyl (-CO(CH₂)₁₆CH₃), or palmitoyl (-CO(CH₂)₁₄CH₃), or the formula (B₁): 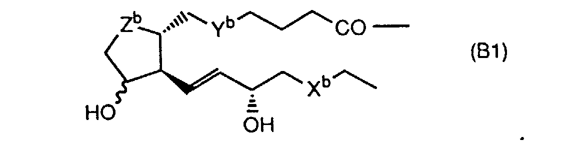 in which X^{b} represents, inter alia, -CH₂CH₂-, Y^{b} represents, inter alia, -CH₂CH₂-, Z^{b} represents, inter alia, -CO-,
and at least one of R^{1b}, R^{2b} and R^{3b} represents the group shown by the formula (B₁), display physiological effects for a prolonged period compared with the prostaglandin itself. In the compounds of the formula (B), the only acyl groups represented by R^{1b}, R^{2b} and R^{3b} are stearoyl and palmitoyl. On the other hand, the corresponding part of the compounds of the present invention of the formula (I) depicted hereinafter, represents C5-13 acyl group, therefore, both of them are different in this regard.

The compounds of the present invention have excellent selectivity and maintenance of action.

Furthermore, liposome formulations of the compounds of the present invention have excellent maintenance and release of active agent.

The present invention accordingly provides a prostaglandin E₁ ester derivative of formula (I): 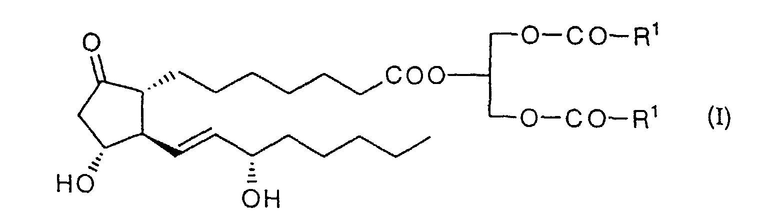 wherein the groups R¹ are the same as each other and are C4-12 alkyl; or a cyclodextrin clathrate thereof.

The present invention also provides a liposome formulation comprising a compound of formula (I) or a cyclodextrin clathrate thereof as an active ingredient;
a process for the preparation of a compound of formula (I) or a cyclodextrin clathrate thereof; and
a pharmaceutical composition comprising a compound of formula (I) or a cyclodextrin clathrate thereof as an active ingredient.

Throughout the specification, it will be understood by those skilled in the at, that all isomers are included in the present invention. For example, alkyl group includes straight-chain and also branched-chain.

In the formula (I), C4-12 alkyl group represented by R¹ means butyl, pentyl, hexyl, heptyl, octyl, nonyl, decyl, undecyl, dodecyl and the isomers thereof.

Preferred R¹ is C4-6 alkyl, or C10-12 alkyl and the isomers thereof. Particularly, preferred R¹ is pentyl, or undecyl.

Cyclodextrin clathrates of the PGE₁ derivatives of the formula (I) may be prepared by the method described in the specification of United States Patent No. 3816393 or 4054736, using α-, β- or γ- cyclodextrins or a mixture thereof.

Converting into their cyclodextrin clathrates serves to increase the stability and solubility in water of the PGE₁ derivatives of the formula (I), and is therefore useful because it facilitates administration as pharmaceuticals.

The compounds of the present invention of formula (I) may be prepared by the elimination of the R² group of a compound of formula (II): 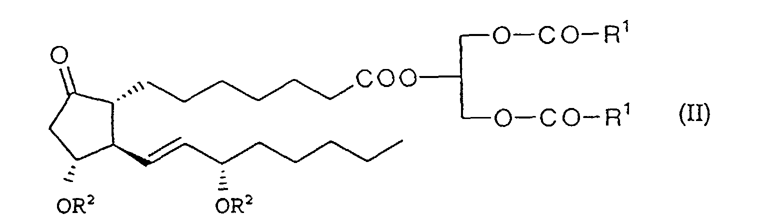 in which R¹ is as hereinbefore defined, and R² is a hydroxyl-protecting group which may be eliminated under acidic conditions, for example tetrahydropyran-2-yl, methoxymethyl or 2-ethoxyethyl. The elimination reaction of the R² group may be carried out in an aqueous solution of organic acid (e.g., acetic acid or p-toluenesulfonic acid) or inorganic acid (e.g., hydrochloric acid or sulfuric acid), in the presence of a water-miscible organic solvent (e.g., a lower alkanol (such as methanol or ethanol) or an ether (e.g., dioxane or tetrahydrofuran)) at a temperature of from room temperature to 75°C. The above-mentioned reaction may preferably be carried out in a mixed solvent comprising acetic acid, water and tetrahydrofuran at a temperature of from 40°C to 50°C.

The compounds of formula (II) may be prepared by the reaction of a compound of formula (III): 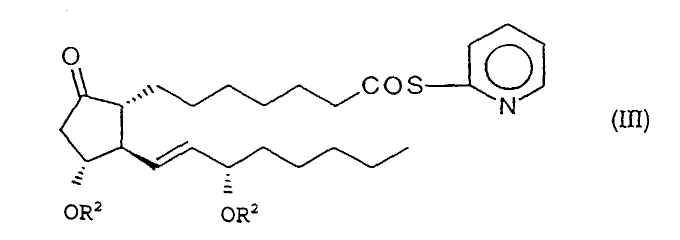 in which R² is as hereinbefore defined, with a compound of formula (IV): 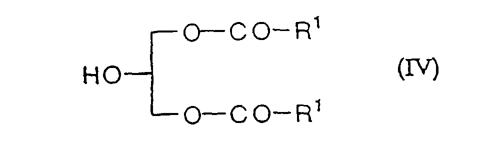 in which R¹ is as hereinbefore defined, without a solvent at a temperature of from 50°C to 100°C.

The compounds of formula (III) may be prepared by the reaction of the free carboxylic acids corresponding to the compounds of formula (II) with 2,2'-dipyridyl disulfide in organic solvent (e.g., acetonitrile) in the presence of triphenylphosphine at from 50°C to the reflux temperature of the solvent.

The compounds of formula (II) may be also prepared by the reaction of the free carboxylic acids corresponding to the compounds of formula (II) with a compound of formula (IV) in an organic solvent (e.g., tetrahydrofuran) in the presence of a tertiary amine (e.g., triethylamine) using a condensing agent (e.g. 1-(3-dimethylaminopropyl)-3-ethylcarbodiimide (hereinafter abbreviated as EDC) or 4-dimethylaminopyridine (hereinafter abbreviated as DMAP)) at a temperature of from 0°C to 40°C.

Of the free carboxylic acids used as starting material corresponding to the compounds of formula (II), (13E)-(11α, 15S)-9-oxo-11,15-bis(tetrahydropyran-2-yloxy)prost-13-enoic acid, for example, is a known compound described in J. Am. Chem. Soc., 92, 2586 (1970).

The other free carboxylic acids corresponding to the compounds of formula (II), and the compounds of formula (IV), are known per se or may be easily prepared from known compounds by methods known per se, e.g., the method described in J. Org. Chem. 35, 2082 (1970).

Concretely, for example, the compounds of formula (IV) may be prepared by the method described in the following Scheme. 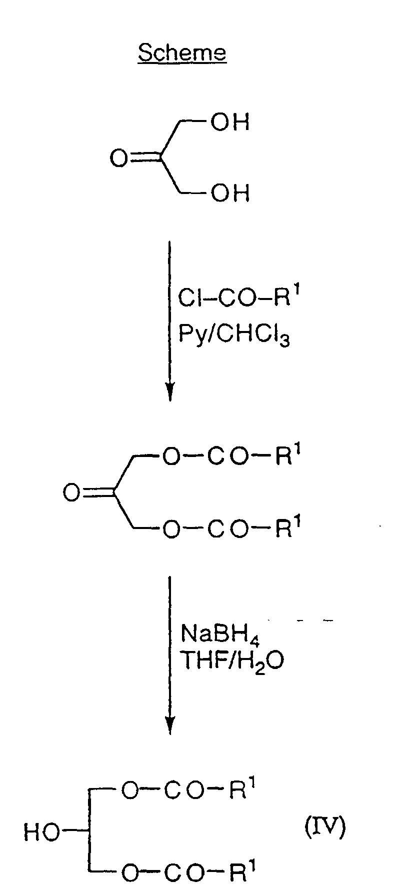 In the above-mentioned Scheme, Py is pyridine,
THF is tetrahydrofuran, and R¹ is as hereinbefore defined.

In spite of having a potent and maintainable blood flow increasing effect, PGE₁ ester derivatives of the formula (I), and cyclodextrin clathrates thereof, have only a weak hypotensive effect, and therefore, may be used as agents for the prevention and/or treatment of peripheral circulatory disorder (e.g., peripheral arterial occlusive disease or thrombotic anginetic obliterence), decubitus, skin ulcers (e.g., ulcers resulting from burns, diabetic ulcers, stenosis of femoral artery and operation stress), and for the maintenance of blood flow after reconstructive vascular surgery.

Blood flow increasing effect and hypotensive effect of the compounds of the present invention, were determined by, for example, the following experiments.

Male rats weighing 200-350g were anesthetized with urethane (25% urethane, 6ml/kg, s.c.). The carotid artery and jugular vein were canulated with polyethylene tubes for measurement of blood pressure and for drug injection, respectively. Blood pressure was obtained with disposable pressure transducer kit (Spectramed, Ltd) and recorded with a recticoder (model RJG-4128, Nihon kohden, Ltd). Also, blood flow was monitored as cutaneous blood flow of dorsum pedis using an attachment-type laser-Doppler flowmeter (model ALF21, Advance, Ltd). Measurements were taken until the values recovered to the level observed before injection of drugs. Injection time was about 10 seconds. The hypotensive effect and blood flow increasing effect were calculated as the maximum hypotensive activity (mmHg) and the area under the curve (AUC) after injection of drugs, respectively.

The results were represented by the dose required to obtain an effective increase in blood flow (main effect), and by the hypotensive effect (side effect) at the same dose.

The compounds of the present invention were administered in the form of liposome formulations (prepared in Example 4). As comparative compound, PGE₁ 1,3-bis(palmitoyloxy)-2-propyl ester described in Example 12 of the specification of Great Britain Patent No. 1322637, was dissolved with β-cyclodextrin as solubilizer, and was administered (solubility of comparative compound was little, so administration without solubilizer was impossible).

The effectiveness of increasing blood flow was determined by the following method. That is to say, it is known that PGE₁ lipid emulsion (on sale) shows efficacy at 5µg/kg i.v. injection in the rat disease model [described in Drug Exp. Clin. Res., 12, 917 (1986)]. In the above-mentioned experimental assessment system for the compounds of the present invention, AUC for PGE₁ lipid emulsion (5µg/kg, i.v.) was 771 on the blood flow increasing effect, so, we used this as an effective value for the increase in blood flow.

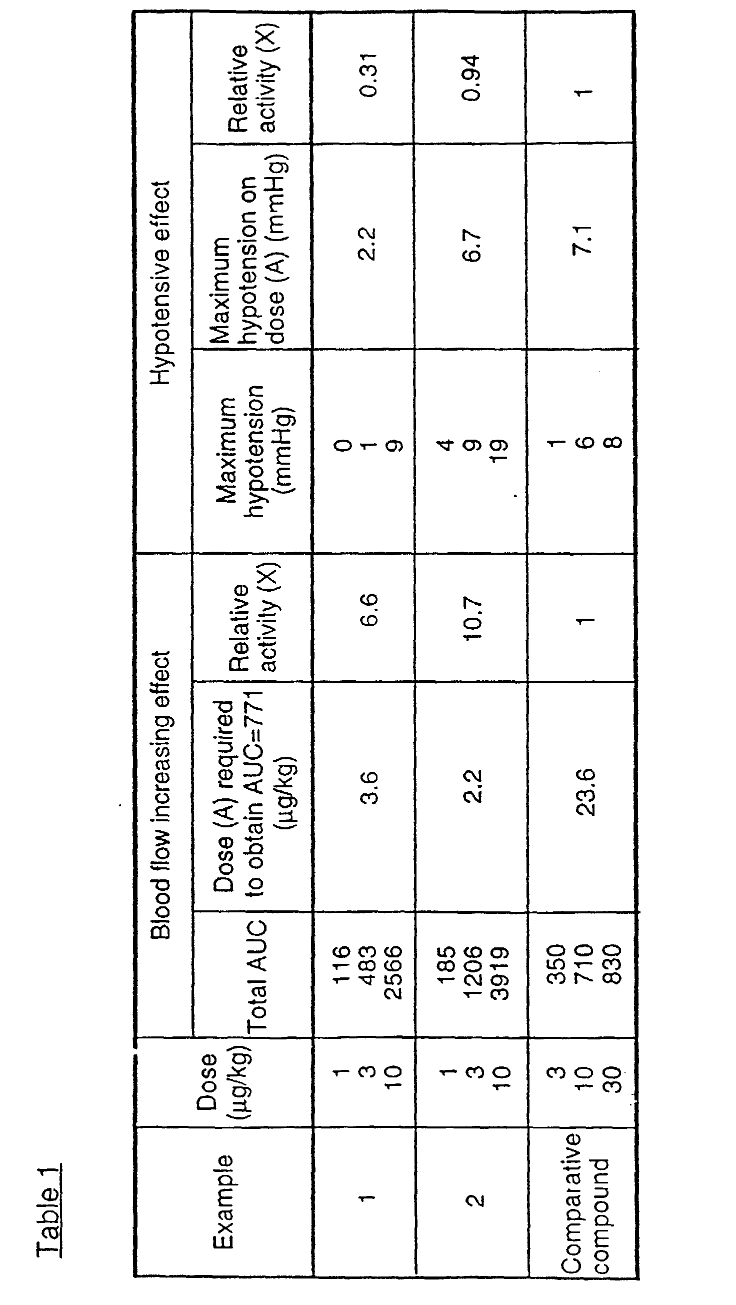

The above Table 1 shows the following facts:
(1) Blood flow increasing effect (main effect) of the compounds of the present invention is about 7 to 11 times better than that of the comparative compound.
(2) On the other hand, hypotensive effect (side effect) of the compounds of the present invention at the effective dose is 0.94 to 0.31 times that of the comparative compound.

It is considered that the compounds of the present invention are significantly better than the comparative compound, if they are used for the agent for the prevention and/or treatment of peripheral circulatory disorder, decubitus, skin ulcers, or for blood flow maintenance after reconstructive vascular surgery.

The toxicity of the compounds of the present invention is very low and therefore the compounds of the present invention may be suitable for pharmaceutical use.

For the purpose above described, the compounds of the formula (I), of the present invention and cyclodextrin clathrates of them may be normally administered systemically or partially, usually by parenteral administration.

The doses to be administered are determined depending upon, for example, age, body weight, symptom, the desired therapeutic effect, the route of administration, and the duration of the treatment. In the human adult, the doses per person are generally from 0.1µg to 500µg, by parenteral administration (preferably intravenous administration), up to several times per day, or continuous administration for from 1 to 24hrs. per day from vein.

As mentioned above, the doses to be used depend upon various conditions. Therefore, there are cases in which doses lower than or greater than the ranges specified above may be used.

The compounds of the present invention may be administered in the form of, for example, injections, liniments or suppositories for parenteral administration.

Injections for parenteral administration include sterile aqueous or non-aqueous solutions, suspensions and emulsions. Aqueous solutions and suspensions may include distilled water for injection or physiological salt solution. Non-aqueous solutions and suspensions may, for example, include propylene glycol, polyethylene glycol, vegetable oil such as olive oil, alcohol such as ethanol or POLYSORBATE80 (registered trade mark).

Injections may comprise additional ingredients other than inert diluents: e.g., preserving agents, wetting agents, emulsifying agents, dispersing agents, stabilizing agents, and assisting agents such as agents to assist dissolution (e.g. glutamic acid or aspartic acid).

They may be sterilized for example, by filtration through a bacteria-retaining filter, by incorporation of sterilizing agents in the compositions or by irradiation. They may also be manufactured in the form of sterile solid compositions which may be dissolved in sterile water or some other sterile diluent(s) for injection immediately before use.

Other compositions for parenteral administration include liquids for external use, and endermic liniments, ointment, suppositories for rectal administration and pessaries for vaginal administration which comprise one or more of the active compound(s) and may be prepared by methods known per se.

Furthermore, the present invention includes the liposome formulations containing PGE₁ ester derivatives of the formula (I) or cyclodextrin clathrates thereof, as active ingredient.

The liposome formulations are uni or multilamellar fine spherical vesicles comprising phosphatidylcholine (e.g., natural phospholipids derived from egg yolk or soya bean, and synthetic phospholipids such as dimyristoylphosphatidylcholine, distearoylphosphatidylcholine, and dipalmitoylphosphatidylcholine) as liposome membrane material. The encapsulation of the drugs into the liposomes makes it possible that the drugs are delivered to the targeting organ, and the release of drugs is prolonged.

Additives other than the active ingredient, which are, for example, sugars (e.g. lactose or mannitol), neutral phospholipids (e.g. cholesterol or triglyceride) or charged lipids (e.g. phosphatidic acid or stearylamine) can be mixed into the liposome formulations.

The liposome formulations may be prepared by methods known per se. For example, suitable methods are described in detail in Liposome Technology Vol. 1, 2 and 3, edited by Gregoriadis., G (published in 1993).

The following Reference Examples and Examples illustrate the present invention, but do not limit the present invention.

The solvents in the parentheses show the developing or eluting solvents and the ratios of the solvents used are by volume in chromatographic separations.

The solvents in the parentheses in NMR show the solvents used for measurement.

### Reference Example 1

1,3-bis(dodecanoyloxy)-propan-2-one 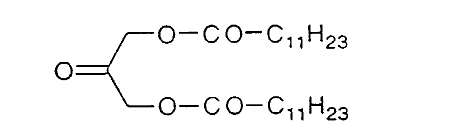 To a suspension of 1,3-propanediol-2-one (2.31g) in chloroform (100ml) was added dodecanoyl chloride (16.5g) and then pyridine (8.5ml)was added dropwise thereto slowly. After stirring for 12 hours at the room temperature, yellowish milky mixture was obtained. This mixture was added to ice water and the solution was extracted with dichloromethane (twice) . The organic layer was washed with a saturated aqueous solution of sodium bicarbonate and a saturated aqueous solution of sodium chloride, successively, dried over magnesium sulfate and evaporated. The yellow solid was dissolved into methanol and impurity was removed by filtration and then filtrate was crystallized to give the title compound (7.3g) as a white crystal having the following physical data.
TLC : Rf 0.55 (n-hexane : ethyl acetate = 4 : 1),
NMR (CDCl₃) : δ 4.75 (4H, s), 2.42 (4H, t, J=7.2Hz), 1.66 (4H, m), 1.27 (32H, m), 0.88 (9H, t, J=7.2Hz).

### Reference Example 2

1,3-bis(dodecanoyloxy)-2-propanol 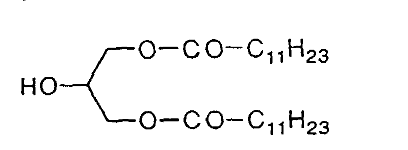 To a solution of 2-oxo derivative (prepared in Reference Example 1 (3.8g)) in tetrahydrofuran (THF) (100ml) was added water (7ml). Sodium borohydride (477mg) was added to the reaction solution at the room temperature and it was stirred for 30min. Ice water was added to the reaction mixture and it was extracted with a mixture solvent of ethyl acetate and n-hexane (1 : 1). The organic layer was washed with a saturated aqueous solution of sodium chloride and dried over magnesium sulfate and evaporated. The residue was purified by silica gel column chromatography (ethyl acetate : n-hexane = 5 : 1) to give the title compound (2.78g) having the following physical data.
TLC : Rf 0.45 (n-hexane : ethyl acetate = 4 : 1),
NMR (CDCl₃) : δ 4.28-4.02 (5H, m), 2.36 (4H, t J=7.0Hz), 1.62 (4H, m), 1.26 (32H, m), 0.88 (9H, t, J=7.2Hz).

### Reference Example 3

(13E)-(11α,15S)-9-oxo-11,15-bis(tetrahydropyran-2-yloxy)-prost-13-enoic acid pyridin-2-ylthio ester 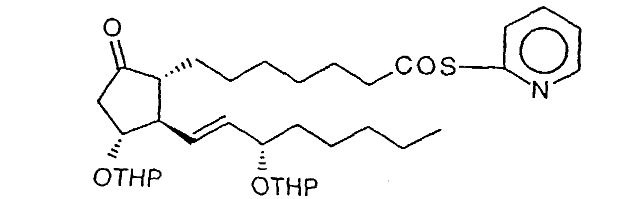 To a solution of (13E)-(11α,15S)-9-oxo-11,15-bis (tetrahydropyran-2-yloxy)prost-13-enoic acid (808mg) in acetonitrile (15ml) was added triphenylphosphine (532mg) and 2,2'-dipyridyl disulfide (448mg), successively, and it was refluxed for 1 hour. The solvent was distilled off and the residue was purified by silica gel column chromatography (n-hexane : ethyl acetate = 2 : 1) to give the title compound (743mg) having the following physical data.
TLC : Rf 0.28 (n-hexane : acetic acid = 1 : 1).

### Example 1

PGE₁ 1,3-bis(dodecanoyloxy)-2-propyl ester 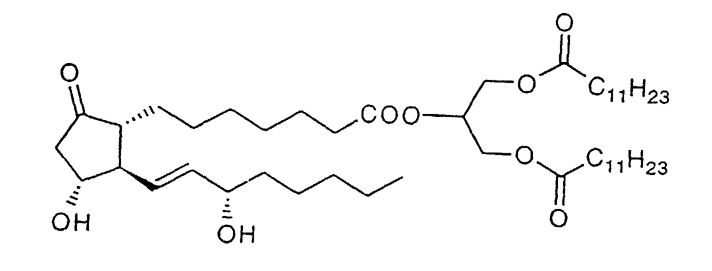 A mixture of the thio ester derivative (prepared in Reference Example 3 (743mg)) and the alcohol derivative (prepared in Reference Example 2 (703mg)) was heated at 70°C-80°C for 18hours without a solvent. After the temperature of the mixture was cooled to room temperature, the mixture was purified by silica gel chromatography (n-hexane : ethyl acetate = 2 : 1) to give PGE₁ 1,3-bis(dodecanoyloxy)-2-propyl ester 11,15-bis (tetrahydropyran-2-yl) ether and the starting material (alcohol derivative) .

The above-mentioned mixture was dissolved into THF (0.5ml) and 87.5% acetic acid (3ml) was added to the solution and the solution was stirred for 2 hours at 80°C. After the temperature of the mixture was cooled to room temperature, water was added thereto, and it was extracted with ethyl acetate. The extract was washed with water, a saturated aqueous solution of sodium bicarbonate and a saturated aqueous solution of sodium chloride, successively, dried over magnesium sulfate and evaporated. The residue was purified by silica gel chromatography (n-hexane : ethyl acetate = 1 : 1→1 : 3) to give the title compound (2.78g) having the following physical data.
TLC : Rf 0.68 (n-hexane : ethyl acetate = 1 : 3),
NMR (CDCl₃) : δ 5.78-5.48 (2H, m), 5.30-5.18 (1H, m), 4.32 (2H, dd, J=12, 6.0Hz), 4.24-4.00 (4H, m), 3.20-2.80 (1H, bs), 2.72 (1H, dd, J=12, 7.5Hz), 2.42-2.15 (9H, m), 2.15-1.90 (2H, m), 1.90-1.48 (10H, m), 1.48-1.18 (42H, m), 1.00-0.90 (9H, m).

### Example 2

PGE₁ 1,3-bis (hexanoyloxy)-2-propyl ester 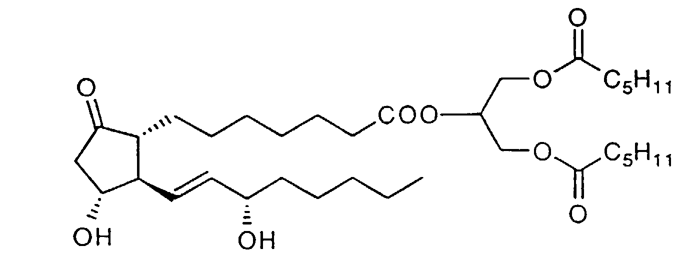 By the same procedures as in Reference Example 1, Reference Example 2 and Example 1 (starting from caproyl chloride instead of dodecanoyl chloride) the compound having the following physical data was obtained.
NMR (CDCl₃) : δ 5.78-5.50 (2H, m), 5.38-5.20 (1H, m), 4.32 (2H, dd, J=12, 4.0Hz), 4.24-4.00 (4H, m), 3.30-2.80 (1H, bs), 2.72 (1H, dd, J=12, 7.5Hz), 2.42-2.15 (7H, m), 2.15-1.90 (2H, m), 1.90-1.48 (8H, m), 1.48-1.18 (22H, m), 1.00-0.90 (9H, m).

### Example 3

Further method of synthesis of PGE₁ 1,3-bis (hexanoyloxy)-2-propyl ester To a solution of (13E)-(11α,15S)-9-oxo-11, 15-bis (tetrahydropyran-2-yloxy)prost-13-enoic acid (2.54g) in THF (5ml) were added 1,3-bis (hexanoyloxy)-2-propanol (2.01g) and EDC (1.43g), successively. Under cooling with ice, to the mixture was added triethylamine (1.6ml) slowly, and furthermore added DMAP (15mg). After the mixture was stirred for 3.5hours at room temperature, water was added to the mixture for stopping the reaction. The reaction mixture was extracted with ethyl acetate. The organic layer was dried over a saturated aqueous solution of sodium chloride and dried over magnesium sulfate and evaporated. The residue was purified by silica gel chromatography (n-hexane : ethyl acetate = 5 : 1) to give the mixture of PGE₁ 1,3-bis (hexanoyloxy)-2-propyl ester 11, 15-bis (tetrahydropyran-2-yloxy) ether and the starting material (alcohol derivative). The above-mentioned mixture was dissolved into THF (3ml) and 87.5% acetic acid (12ml) was added thereto and it was stirred for 2 hours at 80°C. After the temperature of the mixture was cooled to room temperature, water was added thereto, it was extracted with ethyl acetate. The extract was washed with water, a cooled saturated aqueous solution of sodium bicarbonate and a saturated aqueous solution of sodium chloride, successively, dried over magnesium sulfate and evaporated. The residue was purified by silica gel chromatography (n-hexane : ethyl acetate = 1:1→1 :4) to give the title compound (1.62g) having the physical data as described in Example 2.

### Example 4 : Preparation of the liposome formulations

Dimyristoylphosphatidylcholine (hereinafter abbreviated as DMPC, 6mg) and various PGE₁ ester derivatives of the present invention (3-90µg) were dissolved in chloroform and a dry lipid film of them was produced by removing chloroform and standing under reduced pressure for one hour.

The dry lipid film was dispersed into 10% maltose solution (3ml) by using a vortex mixer (type S-100, Taiyokagaku Inc.) and an aqueous suspension (the drug concentrations : 1, 3, 10, 30µg/ml) was obtained. The obtained multilamellar liposome solutions (3ml) were transferred into plastic tubes and were sonicated with a probe sonicator (type : SONIFIER cell disruptor 200, Branson) at the condition of 50% pulsed operation for 15 minutes. The obtained solutions were passed through a membrane filter with pore diameter of 0.2µm to remove titanium particles and liposome formulations with mean diameter of 40-70nm were formed.

### Formulation Example 1 :

The liposome formulations of PGE₁ 1,3-bis(dodecanoyloxy)-2-propyl ester (prepared in Example 4) were divided into 1ml vials and lyophilized for the purposed injection products.

## Claims

1. A prostaglandin E₁ ester derivative of formula (I): 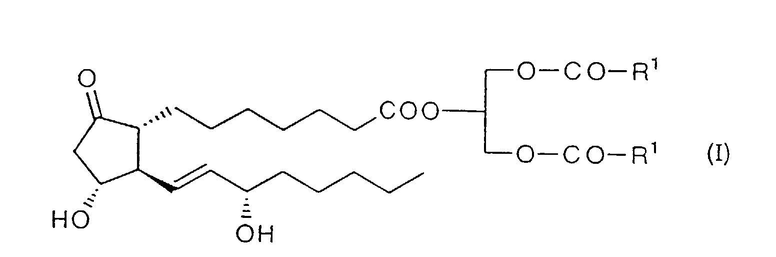 wherein the groups R¹ are the same as each other and are C4-12 alkyl; or a cyclodextrin clathrate thereof.

2. A compound according to claim 1, which is PGE₁ 1,3-bis (hexanoyloxy)-2-propyl ester, or PGE₁ 1,3-bis(dodecanoyloxy)-2-propyl ester.

3. A liposome formulation comprising a prostaglandin E₁ derivative of formula (I) as defined in claim 1 or 2 or a cyclodextrin clathrate thereof, as active ingredient.

4. A liposome formulation according to claim 3, wherein the liposome membrane material comprises dimyristoylphosphatidylcholine.

5. A liposome formulation according to claim 3, wherein the liposome membrane material comprises egg lecithin.

6. A liposome formulation according to one of any claims 3 to 5, which is obtainable by freeze-drying.

7. A process for the preparation of a compound of formula (I): 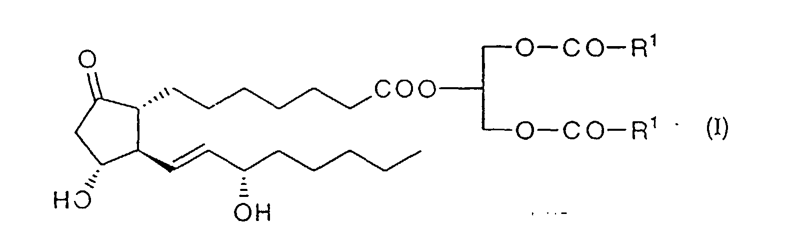 wherein R¹ is as defined in claim 1, or a cyclodextrin clathrate thereof, which comprises elimination of the R² group of a compound of formula (II) : 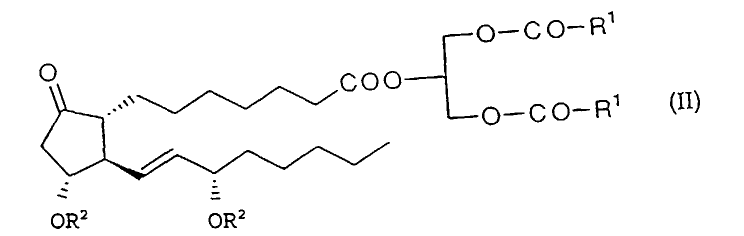 wherein R¹ is as hereinbefore defined and R² is a hydroxyl-protecting group which may be eliminated under acidic conditions, and, if desired, conversion of the compound of formula (I) thus obtained into a cyclodextrin clathrate thereof.

8. A pharmaceutical composition which comprises a prostaglandin E₁ derivative of formula (I) as defined in claim 1 or 2 or a cyclodextrin clathrate thereof, as active ingredient, and a pharmaceutically acceptable carrier.

9. Use of a prostaglandin E₁ derivative of formula (I) as defined in claim 1 or 2 or a cyclodextrin clathrate thereof in the manufacture of a medicament for the prevention and/or treatment of peripheral circulatory disorder, decubitus, or skin ulcers.

10. Use of a prostaglandin E₁ derivative of formula (I) as defined in claim 1 or 2 or a cyclodextrin clathrate thereof in the manufacture of a medicament for blood flow maintenance after reconstructive vascular surgery.

## Patentansprüche

1. Prostaglandin E₁-Esterderivat der Formel (I): 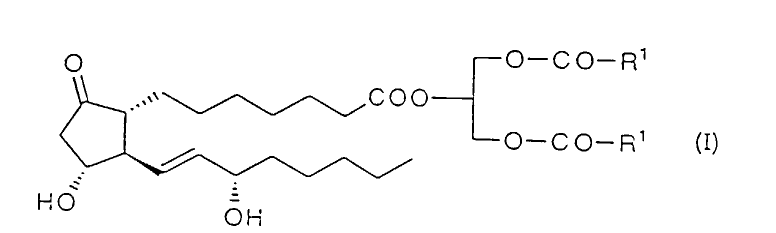 worin die Gruppen R¹ gleich sind und für C4-12 Alkyle stehen, oder ein Cyclodextrinclathrat hiervon.

2. Verbindung nach Anspruch 1, nämlich PGE₁ 1,3-bis(hexanoyloxy)-2-propylester oder PGE₁ 1,3-bis(dodecanoyloxy)-2-propylester.

3. Liposomenrezeptur, umfassend ein Prostaglandin-E₁-Derivat der Formel (I) entsprechend Anspruch 1 oder 2 oder ein Cyclodextrinclathrat hiervon als aktiven Bestandteil.

4. Liposomenrezeptur nach Anspruch 3, wobei das Liposomenmembranmaterial Dimyristoylphosphatidylcholin umfaßt.

5. Liposomenrezeptur nach Anspruch 3, wobei das Liposomenmembranmaterial Eilecithin umfaßt.

6. Liposomenrezeptur nach einem der Ansprüche 3 bis 5, erhältlich durch Gefriertrocknen.

7. Verfahren zur Herstellung einer Verbindung der Formel (I): 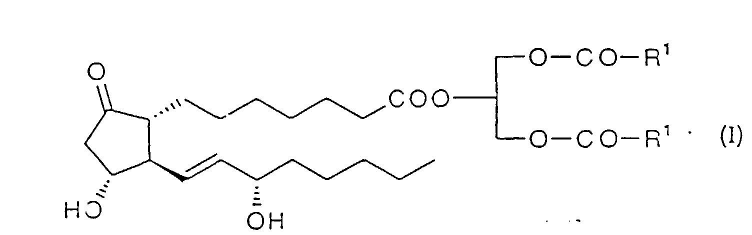 worin R¹ die in Anspruch 1 angegebene Bedeutung besitzt, oder eines Cyclodextrinclathrats hiervon durch Eliminieren der Gruppe R² einer Verbindung der Formel (II) : 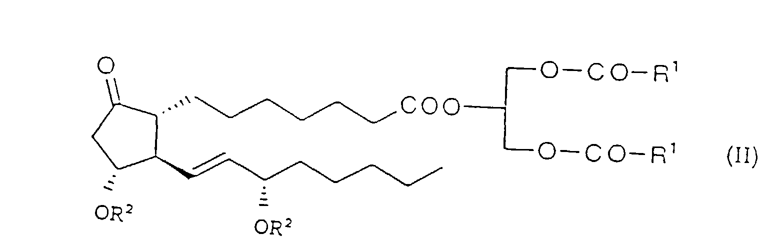 worin R¹ die zuvor angegebene Bedeutung besitzt und R² für eine unter sauren Bedingungen eliminierbare Hydroxyschutzgruppe steht, und gewünschtenfalls Umwandeln der hierbei erhaltenen Verbindung der Formel (I) in ein Cyclodextrinclathrat hiervon.

8. Arzneimittelzubereitung, umfassend ein Prostaglandin-E₁-Derivat der Formel (I) gemäß Anspruch 1 oder 2 oder ein Cyclodextrinclathrat hiervon als aktiven Bestandteil und einen pharmazeutisch akzeptablen Träger.

9. Verwendung eines Prostaglandin-E₁-Derivats der Formel (I) gemäß Anspruch 1 oder 2 oder eines Cyclodextrinclathrats hiervon bei der Herstellung eines Medikaments zur Verhinderung und/oder Behandlung von peripheren Kreislaufstörungen, Decubitus oder Hautgeschwüren.

10. Verwendung eines Prostaglandin-E₁-Derivats der Formel (I) gemäß Anspruch 1 oder 2 oder eines Cyclodextrinclathrats hiervon bei der Herstellung eines Medikaments zur Blutstromerhaltung nach wiederherstellender Gefäßchirurgie.

## Revendications

1. Ester de prostaglandine E₁ de formule (I): 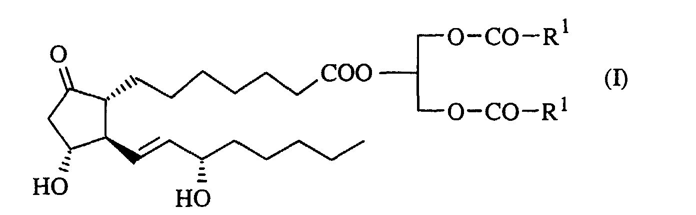 dans lequel les deux groupes R¹ sont identiques et sont des groupes alkyle en C₄₋₁₂ ; ou un de ses clathrates de cyclodextrine.

2. Composé selon la revendication 1, qui est l'ester de 1,3-bis(hexanoyloxy)-2-propyle de la PGE₁ ou l'ester de 1,3-bis(dodécanoyloxy)-2-propyle de la PGE₁.

3. Formulation de liposomes comprenant un dérivé de prostaglandine E₁ de formule (I) tel que défini dans la revendication 1 ou 2 ou un de ses clathrates de cyclodextrine en tant que principe actif.

4. Formulation de liposomes selon la revendication 3, dans laquelle la matière constituant la membrane des liposomes comprend de la dimyristoylphosphatidylcholine.

5. Formulation de liposomes selon la revendication 3, dans laquelle la matière constituant la membrane des liposomes comprend de la lécithine d'oeuf.

6. Formulation de liposomes selon une quelconque des revendications 3 à 5, que l'on peut obtenir par lyophilisation.

7. Procédé pour la préparation d'un composé de formule (I): 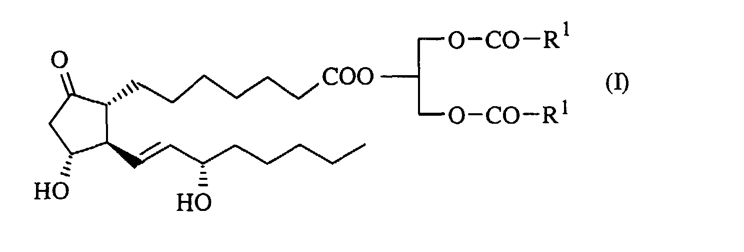 dans lequel R¹ est tel que défini dans la revendication 1, ou d'un de ses clathrates de cyclodextrine, comprenant l'élimination du groupe R² d'un composé de formule (II) : 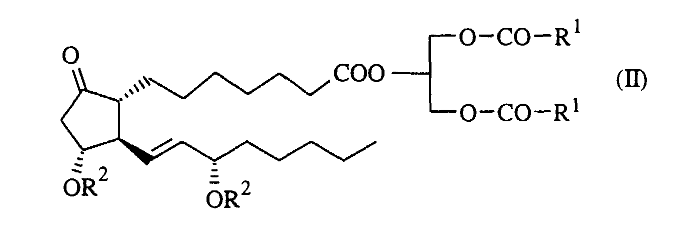 dans laquelle R¹ est tel que défini ci-dessus et R² est un groupe protecteur d'hydroxyle pouvant être éliminé en conditions acides, et, si on le désire, la conversion du composé de formule (I) ainsi obtenu en un de ses clathrates de cyclodextrine.

8. Composition pharmaceutique comprenant un dérivé de prostaglandine E₁ de formule (I) tel que défini dans la revendication 1 ou 2 ou un de ses clathrates de cyclodextrine, en tant que principe actif et un support pharmaceutiquement acceptable.

9. Utilisation d'un dérivé de prostaglandine E₁ de formule (I) tel que défini dans la revendication 1 ou 2 ou d'un de ses clathrates de cyclodextrine dans la fabrication d'un médicament pour la prévention et/ou le traitement des troubles circulatoires périphériques, des escarres de décubitus ou des ulcères cutanés.

10. Utilisation d'un dérivé de prostaglandine E₁ de formule (I) tel que défini dans la revendication 1 ou 2 ou d'un de ses clathrates de cyclodextrine dans la fabrication d'un médicament pour le maintien du débit sanguin après une intervention chirurgicale vasculaire reconstructive.
